(19) Europäisches Patentamt
European Patent Office
Office européen des brevets

(11) **EP 4 173 555 A1**

(12) **EUROPEAN PATENT APPLICATION**

(43) Date of publication:
**03.05.2023 Bulletin 2023/18**

(21) Application number: **21204644.5**

(22) Date of filing: **26.10.2021**

(51) International Patent Classification (IPC):
***A61B 5/021*** (2006.01)    ***A61B 5/022*** (2006.01)
***A61B 5/00*** (2006.01)

(52) Cooperative Patent Classification (CPC):
**A61B 5/02125; A61B 5/0225; A61B 5/02416;**
**A61B 5/0245; A61B 5/7278;** A61B 5/02225

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
• **SCHMITT, Lars**
  **Eindhoven (NL)**
• **MUEHLSTEFF, Jens**
  **Eindhoven (NL)**
• **BRESCH, Erik**
  **Eindhoven (NL)**

(74) Representative: **Philips Intellectual Property &**
**Standards**
**High Tech Campus 52**
**5656 AG Eindhoven (NL)**

(54) **DEVICE, SYSTEM AND METHOD FOR CALIBRATING A BLOOD PRESSURE SURROGATE FOR USE IN MONITORING A SUBJECT'S BLOOD PRESSURE**

(57)    The present invention relates to a device, system and method for calibrating a blood pressure, BP, surrogate for use in monitoring a subject's blood pressure. The device comprises a BP input (51), a sensor input (52) and a processing unit (53) that is configured to compute a control signal for controlling the pressure-delivery system (10) to repeatedly inflate its cuff in partial inflations up to a cuff pressure below the subject's systolic BP, compute pulse-related values from the first and second time-dependent sensor signals, measured during the repeated partial inflations of the cuff, using a first feature in the first time-dependent sensor signal and a second feature in the second time-dependent sensor signal, the pulse-related values being pulse arrival time, PAT, values or pulse transit time, PTT, values, and compute a calibration parameter for a BP surrogate from the BP measurement value and pairs of the pulse-related values and corresponding cuff pressure values, computed from the first and second time-dependent sensor signals measured during the repeated partial inflations of the cuff, a pair comprising a pulse-related value and a corresponding, temporally related cuff pressure value.

FIG.18

**Description**

FIELD OF THE INVENTION

The present invention relates to a device, system and method for calibrating a blood pressure (BP) surrogate for use in monitoring a subject's blood pressure.

BACKGROUND OF THE INVENTION

A general trend for hemodynamic measurements in acute care is to become more continuous, less invasive, and less obtrusive. Blood pressure (BP) is a basic hemodynamic parameter used in all hospital settings to assess health status of a patient. Arterial Blood Pressure (ABP) is a key physiological parameter relevant for medical diagnosis, prevention as well as therapy guidance. Invasive measurements - the Gold Standard - provide the most accurate continuous measurements, but they can be done by trained medical staff only, and they are applied mostly in high acuity settings, which require real-time alarming and very close monitoring.

The established way to measure blood pressure non-invasively is by means of an upper arm cuff (NIBP = non-invasive blood pressure). Although being practical, a NIBP measurement is intermittent only and therefore needs to be repeatedly conducted (typically automatically) in monitoring applications, e.g. every few minutes during surgery, or in ICU typically every 15 min. Most NIBP measurements in patient monitoring are being implemented by standard cuff-based ABP measurements using automated oscillometry providing intermittent measurements. There is an unmet need in continuous and, at the same time, accurate non-invasive blood pressure measurement technologies. It would be a key discriminator to have continuous non-invasive blood pressure information available in-between blood pressure measurement intervals, in order to not miss hypotensive (or hypertensive) episodes which are associated with adverse patient outcome.

An elegant method to achieving this is to utilize continuous physiological signals that are already available in acute care settings and to infer continuous blood pressure information (a continuous BP surrogate parameter) from those signals. Therefore, additional costs could be kept minimum and clinical workflow would remain unaffected.

Several dedicated devices exist for continuous NIBP measurements, such as e.g. finger-cuff devices. However, none of those have seen widespread hospital adoption yet. This is due to various issues related to lack of accuracy and robustness, but also due to the additional costs and the adverse effect on clinical workflow.

An approach for continuous non-invasive blood pressure measurements is based on BP surrogates. Surrogates are well-known parameters such as e.g. the pulse transit time (PTT) and pulse arrival time (PAT), pulse wave velocity or combinations thereof. With these parameters blood pressure can be inferred non-invasively and continuously without using external pressures except for reference or initialization purposes, often called calibration. Calibration is a key issue for practical applications in clinical and home settings.

US 2010/160798 A1 discloses a technique for continuous measurement of BP based on PTT, which does not require any external calibration. This technique is carried out with a body-worn monitor that measures BP and other vital signs, and wirelessly transmits them to a remote monitor. A network of body-worn sensors, typically placed on the patient's right arm and chest, connect to the monitor and measure time-dependent ECG, PPG, accelerometer, and pressure waveforms. The sensors can include a cuff that features an inflatable bladder coupled to a pressure sensor, three or more electrical sensors (e.g. electrodes), three or more accelerometers, a temperature sensor, and an optical sensor (e.g., a light source and photodiode) attached to the patient's thumb. A dedicated PTT change is achieved during a controlled decrease in transmural pressure under the cuff.

SUMMARY OF THE INVENTION

It is an object of the present invention to provide a device, system and method enabling a further improvement in the accuracy of continuous non-invasive BP measurements using BP surrogates.

In a first aspect of the present invention a device for calibrating a BP surrogate for use in monitoring a subject's blood pressure is presented, the device comprising:

a BP input configured to obtain time-dependent cuff pressure values during inflation of a cuff of a pressure-delivery system attached to a subject's body part and a BP measurement value;
a sensor input configured to obtain a first time-dependent sensor signal and a second time-dependent sensor signal, both related to the subject's heartbeat and measured at different sites of the subject's body during the inflation of the cuff; and
a processing unit configured to:

- compute a control signal for controlling the pressure-delivery system to repeatedly inflate its cuff in partial inflations up to a cuff pressure below the subject's systolic BP,
- compute pulse-related values from the first and second time-dependent sensor signals, measured during the repeated partial inflations of the cuff, using a first feature in the first time-dependent sensor signal and a second feature in the second time-dependent sensor signal, the pulse-related values being pulse arrival time, PAT, values or pulse transit time, PTT, values, and
- compute a calibration parameter for a BP surrogate from the BP measurement value and pairs of the pulse-related values and corresponding cuff pressure values, computed from the first and second time-dependent sensor signals measured during the repeated partial inflations of the cuff, a pair comprising a pulse-related value and a corresponding, temporally related cuff pressure value.

In a further aspect of the present invention a system for calibrating a BP surrogate for use in monitoring a subject's blood pressure is presented, the system comprising:

a pressure-delivery system comprising a cuff configured to be attached to the subject's body part and to deliver a pressure to the subject's body part by inflating the cuff;
a pressure sensor configured to acquire time-dependent cuff pressure values during inflation of a cuff of the pressure-delivery system attached to the subject's body part and to acquire or infer a BP measurement value;
a first sensor configured be attached to a first site of the subject's body and to acquire a first time-dependent sensor signal related to the subject's heartbeat during the inflation of the cuff;
a second sensor configured be attached to a second site of the subject's body and to acquire a second time-dependent sensor signal related to the subject's heartbeat during the inflation of the cuff; and
a device as disclosed herein for computing one or more calibration parameters for calibrating a BP surrogate from the BP measurement value, the time-dependent cuff pressure values and the first and second time-dependent sensor signals.

In yet further aspects of the present invention, there are provided a corresponding method, a computer program which comprises program code means for causing a computer to perform the steps of the method disclosed herein when said computer program is carried out on a computer as well as a non-transitory computer-readable recording medium that stores therein a computer program product, which, when executed by a processor, causes the method disclosed herein to be performed.

Preferred embodiments of the invention are defined in the dependent claims. It shall be understood that the claimed method, system, computer program and medium have similar and/or identical preferred embodiments as the claimed system, in particular as defined in the dependent claims and as disclosed herein.

The present invention is based on the finding that changes in PAT (or PTT) during cuff pressurization are distorted by dynamic filling effects in the lower arm (or, more generally, in a peripheral region of the subject's body part at which the cuff is arranged) due to venous occlusion. This finding affects the way how to obtain the one or more calibration parameters. For instance, parameter regression - as one preferred option to compute the one or more calibration parameters - is affected, which needs to be addressed for the inference of a sensitivity parameter. The effect has been observed and interpreted from invasively measured BP signals acquired peripheral to the cuff, e.g. in the lower arm when the cuff is arranged at the upper arm, or in the lower leg when the cuff is arranged at the upper leg.

According to the present invention only low cuff pressure levels are used for calibration, i.e. the cuff is only partly inflated for data acquisition. Repeated sampling of those relevant cuff pressure levels is feasible in a short enough time period, such that the BP level is stable and does not change, which would distort the calibration process. Furthermore, repeated inflation ramps (with or even without a pause in-between) are enabled by low maximum pressure: there is no need to wait for tissue and arteries to recover from high pressurization (i.e., there is no hysteresis effects).

Low cuff pressure levels, especially cuff pressure levels below diastolic pressure, are very unobtrusive for the patient, particularly for awake patients, resulting in less discomfort and less stress to skin than full inflation ramps up to supra-systolic levels. Another advantage of the present invention is that it does not pose extra requirements on the pressure-delivery system of a NIBP device.

The BP measurement value used for the calibration (i.e. for computing the calibration parameter) may be acquired through BP measurement from a BP measurement device.

There are different embodiments how to control the pressure-delivery system in the repeated partial inflations. They all try to further avoid the negative dynamic filling effects mentioned above and thus contribute to improving accuracy of the calibration and thus finally improve BP determination by use of the calibrated BP surrogate.

In an embodiment the processing unit is configured to compute the control signal for controlling the pressure-delivery system to inflate its cuff in the partial inflations as long as a peripheral BP is substantially constant, in particular varies by less than 10 percent or less than 5 percent. Other reasonable values may be used as well.

The processing unit may hereby be configured to determine if the peripheral BP is substantially constant based on the first and/or second time-dependent sensor signal, in particular by determining the period during which an amplitude of the second time-dependent signal is substantially constant, in particular varies by less than 10 percent or less than 5 percent (or any other reasonable threshold that is predetermined or set by user) with respect to a previously acquired average value. This previously acquired average value refers to a previous average value of the second time-dependent signal.

According to another embodiment the processing unit is configured to compute the control signal for controlling the pressure-delivery system to inflate its cuff in the partial inflations up to the subject's diastolic BP, in particular the subject's latest measured diastolic BP, up to the subject's mean BP, in particular the subject's latest measured mean BP, or up to a set threshold BP. This enables a rather simple but efficient implementation of the claimed disclosed solution.

The processing unit may be configured to compute the control signal for controlling the pressure-delivery system to inflate its cuff in the partial inflations up to a cuff pressure at which the PAT exceeds a PAT threshold, in particular an absolute PAT threshold or a relative PAT threshold compared to a baseline PAT. Hereby, the processing unit may be configured to compute the baseline PAT by averaging PAT measurements over a period, in particular a period in the range of 10 s to 5 min (other reasonable values may be possible as well). A reasonable threshold for a relative change, i.e. PAT compared to baseline PAT, may for instance be in the range of 10 to 60 ms, e.g. 30 ms.

In another embodiment the processing unit is configured to compute the control signal for controlling the pressure-delivery system to inflate its cuff in the partial inflations up to a cuff pressure at which an amplitude of the second time-dependent signal exceeds an amplitude threshold, in particular an absolute amplitude threshold or a relative amplitude threshold. Such criteria can be easily set by a user or predetermined in advance. A reasonable threshold for a relative change may for instance be in the range of 5 to 20%, e.g. 10%.

The processing unit is configured to compute the control signal for controlling the pressure-delivery system to inflate its cuff in the partial inflations at a predetermined inflation speed or an inflation speed dependent on one or more of the subject's heart rate, the subject's diastolic BP, the subject's mean BP, and the subject's systolic BP.

In practical embodiments the processing unit may be configured to:

- compute the control signal for controlling the pressure-delivery system to deflate its cuff after each iteration of a partial inflation, and
- i) compute, for each iteration, the one or more calibration parameters from pairs of pulse-related values and corresponding cuff pressure values obtained in the respective iteration using separate regressions, and average the respective calibration parameters computed for the two or more iterations into one or more averaged calibration parameters for use by the BP surrogate, or

 ii) compute the one or more calibration parameters from pairs of pulse-related values and corresponding cuff pressure values obtained in the two or more iterations in a single regression.

Both options i) and ii) may lead to an increased accuracy depending on the situation and application of the device and method of the present invention. It should be noted that the applied cuff pressure induces a transmural pressure change under the cuff.

In another embodiment the processing unit is configured to compute the control signal for controlling the pressure-delivery system to inflate its cuff in a full inflation beyond the subject's systolic BP before and/or after one or more iterations of partial inflation to obtain a time-dependent BP reference measurement for use in monitoring the subject's BP using the BP surrogate. The BP value measured last may particularly be used in the computation of the calibration parameter.

The processing unit may further be configured to control how many iterations of partial inflation are conducted based on a comparison of a regression error to a regression error threshold. Further, it may be configured to control if and when a full inflation is conducted according to a fixed or variable schedule or if one or more calibration parameters substantially changed in the last computation, in particular changed by more than 10 percent (or more than 15 or 20 percent). Thus, a tradeoff may be made between additional measurement time / effort and increased accuracy of the calibration.

Still further, the processing unit may be configured to determine BP values when no pressure is delivered to the subject's body part by the pressure-delivery system by use of the computed BP surrogate and the measured first and second time-dependent sensor signals measured at different sites of the subject's body when no pressure is delivered to the subject's body part. This enables unobtrusive determination of the subject's BP without inflation of the cuff, i.e., the subject's BP can be continuously monitored without disturbing or harming the patient due to the cuff inflation.

In preferred embodiments the first time-dependent sensor signal is an ECG signal and/or the second time-dependent sensor signal is a photoplethysmography, PPG, signal, in particular a contact PPG signal or a remote PPG signal. Other signals that are related to the subject's heart rate and allow the computation of PAT and/or PTT can be used in addition or as alternative. ECG and PPG signals are commonly known signals from which PAT and/or PTT can be computed in a generally known manner that can be used by the device and method of the present invention as well.

The claimed system may further comprise a control unit configured to control a pressure-delivery system, e.g. based on a control signal provided by the device or computed by the system. The control unit may be part of the device or may be an external entity.

The present invention may also be used in combination with one or more of the following embodiments.

In an embodiment the processing unit is configured to:

- compute, during inflation of the cuff, pulse-related values from the first and second time-dependent sensor signals using a first feature in the first time-dependent sensor signal and a second feature in the second time-dependent sensor signal, the pulse-related values being pulse arrival time, PAT, values or pulse transit time, PTT, values, and

- compute a calibration parameter for a BP surrogate from the BP measurement value and pairs of pulse-related values and corresponding cuff pressure values, a pair comprising a pulse-related value and a corresponding, temporally related cuff pressure value wherein only pairs of pulse-related values and corresponding cuff pressure values are used for computing the BP surrogate for which one or more predetermined criteria with respect to BP and/or cuff pressure are fulfilled.

This embodiment enables reliable personalized calibration procedures (sometimes also called initiation or initialization procedures) utilizing cuff inflations to induce well-defined associated changes in blood pressure surrogates. Insights on physiological effects during cuff inflation resulted in optimized methods and procedures used for computing the one or more calibration parameters. The reliably calibrated blood pressure surrogates enable accurate non-invasive tracking of blood pressure, i.e., by use of the calibrated BP surrogate an - initially open / undefined - set of parameter can be translated into the meaningful clinical parameter blood pressure.

One or more conditions (or criteria) are used according to embodiments to select pairs of pulse-related values and corresponding cuff pressure values that are then used for computing a calibration parameter for a BP surrogate to infer BP. These conditions relate to BP and/or cuff pressure, i.e., depending on BP and/or cuff pressure it is decided if a pair of a pulse-related value and a corresponding cuff pressure value will be used for computing a calibration parameter for a BP surrogate or not. Multiple embodiments for those conditions exist as will be explained in detail in the following.

According to an embodiment the processing unit is configured to use only pairs of pulse-related values and corresponding cuff pressure values for computing the calibration parameter for the BP surrogate that have been computed from the first and second time-dependent sensor signals measured during a partial inflation of the cuff. It has been found that at high cuff pressure no reliable pulse signals due to pulse distortions appear in peripheral part of the body part at which the cuff is arranged, which may negatively affect the computation of the calibration parameter if pairs of values are used that are computed from sensor signals measured during a fully or almost fully inflated cuff. These negative effects are avoided by this embodiment since only pulse signals with defined relationships to BP are taken into account, which will improve accuracy of the calibration and thus finally improve BP determination by use of the calibrated BP surrogate.

According to another embodiment the processing unit is configured to use only pairs of pulse-related values and corresponding cuff pressure values for computing the calibration parameter for the BP surrogate for which a peripheral BP is substantially constant, in particular varies by less than 10 % or less than 5 %. Other reasonable values may be used as well. Similar effects can be achieved in this way as explained above, although a different condition is used for selecting pairs of values for the computation.

The processing unit may hereby be configured to determine if the peripheral BP is substantially constant based on the first and/or second time-dependent sensor signal. For instance, the period during which an amplitude of the second time-dependent signal is substantially constant, in particular varies by less than 10 % or less than 5 % (or any other reasonable threshold that is predetermined or set by user) with respect to a previously acquired average value, may be determined for this purpose. This previously acquired average value refers to a previous average value of the second time-dependent signal.

The processing unit may further be configured to use only pairs of pulse-related values and corresponding cuff pressure values for computing the calibration parameter for the BP surrogate for which the cuff pressure value is below the subject's diastolic BP, in particular the subject's latest measured diastolic BP, or below the subject's mean BP, in particular the subject's latest measured mean BP, or below a set threshold cuff pressure. This enables a rather simple but efficient implementation of the disclosed solution.

In another embodiment the processing unit is configured to use only pairs of pulse-related values and corresponding cuff pressure values for computing the calibration parameter for the BP surrogate for which the cuff pressure value is in a range between a set minimum cuff pressure and a set maximum cuff pressure, in particular wherein the minimum cuff pressure is set in the range of 10 to 30 mmHg and the maximum cuff pressure is set in the range of 40 to 90 mmHg (or in a range defined by other reasonable values). This enables a rather simple but efficient implementation of the disclosed solution as well.

In a still further embodiment, the processing unit may be configured to use only pairs of pulse-related values and corresponding cuff pressure values for computing the calibration parameter for the BP surrogate for which the pulse-related values are substantially constant, in particular vary by less than 10 % (or any other reasonable value), or until the slope of a curve of the pulse-related values over time exceeds a threshold. These embodiments make use of other conditions that can be easily implemented.

The processing unit may further be configured to compute a control signal for controlling the pressure-delivery system to inflate its cuff, in particular to fully inflate the cuff for acquisition of the BP measurement value and to only partly inflate the cuff for acquisition of the first time-dependent sensor signal and the second time-dependent sensor signal. This enables the device to actively control the inflations of the cuff as needed for either a BP measurement or for the acquisition of sensor signals used for determining the calibration parameter. The BP measurement value used for the calibration (i.e. for computing the calibration parameter) may be acquired through BP measurement from a BP measurement device.

In preferred embodiments the first time-dependent sensor signal is an ECG signal and/or the second time-dependent sensor signal is a photoplethysmography, PPG, signal, in particular a contact PPG signal or a remotely acquired PPG signal (e.g. acquired by a camera as used in remote PPG). Other signals that are related to the subject's heart rate and allow the computation of PAT and/or PTT can be used in addition or as alternative. For instance, bio-impedance or cuff signals may be used instead of PPG signals. ECG and PPG signals are commonly known signals from which PAT and/or PTT can be computed in a generally known manner that can be used by the disclosed device and method as well.

It is further preferred in an embodiment that the processing unit is configured to compute the calibration parameter by use of a regression. For instance, the calibration parameter may be computed as a slope of a dependency of a relation of the blood pressure as a function of the blood pressure surrogate for zero cuff pressure. Via regression a parameterized functional relationship of the transmural blood pressure and the BP surrogate is derived, which serve as transfer function to infer blood pressure from the BP surrogate when the cuff is not inflated (zero cuff pressure). More details are e.g. described in Pielmus, A. G., Miihlsteff, J., Bresch, E., Glos, M., Jungen, C., Mieke, S., Zaunseder, S. (2021). Surrogate based continuous noninvasive blood pressure measurement. Biomedical Engineering/Biomedizinische Technik, 66(3), 231-245.

Still further, the processing unit may be configured to determine BP values when no pressure is delivered to the subject's body part by the pressure-delivery system by use of the computed BP surrogate and the measured first and second time-dependent sensor signals measured at different sites of the subject's body when no pressure is delivered to the subject's body part. This enables unobtrusive determination of the subject's BP without inflation of the cuff, i.e., the subject's BP can be continuously monitored without disturbing or harming the patient due to the cuff inflation.

BRIEF DESCRIPTION OF THE DRAWINGS

These and other aspects of the invention will be apparent from and elucidated with reference to the embodiment(s) described hereinafter. In the following drawings:

Fig. 1 shows a schematic diagram of an embodiment of the general concept of the present invention.

Fig. 2 shows a diagram of an ECG signal and a PPG signal in which PAT is indicated.

Fig. 3 shows diagrams illustrating robust versus non-robust regression in case of a linear model.

Fig. 4 shows a diagram of an example of induced PAT increase during a cuff-inflation.

Fig. 5 shows a diagram illustrating the general concept of calibration.

Fig. 6 shows a schematic diagram of a setup used in the general concept to obtain invasively measured BP signals acquired peripheral to the cuff.

Fig. 7 shows diagrams of signals measured by the setup shown in Fig. 5.

Fig. 8 shows an analysis of inflation processes with impact on the blood pressure measured peripherally behind the cuff.

Fig. 9 shows diagram of transmural blood pressure vs. measured PAT.

Fig. 10 shows a diagram of an embodiment of a system according to the present invention.

Fig. 11 shows a diagram of an embodiment of a device according to the present invention.

Fig. 12 shows a diagram of an embodiment of a method according to the present invention.

Fig. 13 shows a diagram of PAT values versus $P_{cuff}$ values during a complete cuff inflation process.

Fig. 14 shows another embodiment of a method using the present invention.

Fig. 15 shows diagrams illustrating that $P_{cuff}$ / PAT value pairs are only used as long as a feature in the PPG signal indicates no pulse pressure change in the peripheral arm blood pressure.

Fig. 16 shows another embodiment of a method using the present invention.

Fig. 17 shows diagram illustrating an example for fitting a parameterized model to measured data pairs obtained during a full inflation process and during a partial inflation.

Fig. 18 shows a diagram of another embodiment of a method according to the present invention.

Fig. 19 shows a diagram illustrating an embodiment of a cuff pressure profile including two partial inflations and a subsequent full inflation.

Fig. 20 shows a diagram illustrating an embodiment of a cuff pressure profile including a full inflation and two subsequent partial inflations.

Fig. 21 shows a diagram illustrating an embodiment of a cuff pressure profile that provides continual tracking of recalibration need through repeated partial inflations.

DETAILED DESCRIPTION OF THE INVENTION

The present invention is related to the development of an ABP measurement technology to enable accurate continuous blood pressure monitoring. The BP tracking in between the intervals of standard cuff-based non-invasive intermittent blood pressure measurements is based on recalibrated/initialized blood pressure surrogates. The calibration is done using the NIBP measurements themselves (Systolic (SBP), Diastolic (DBP) and mean (MBP) blood pressure) as well as the NIBP measurement inflation/deflation processes, which induce controlled BP-related disturbances of the surrogate signals. The surrogates are obtained from features in signals related to the subject's heartbeat such as the electrocardiogram (ECG) or photo-plethysmogram (PPG) or via their combination. These signals are acquired in these setting routinely.

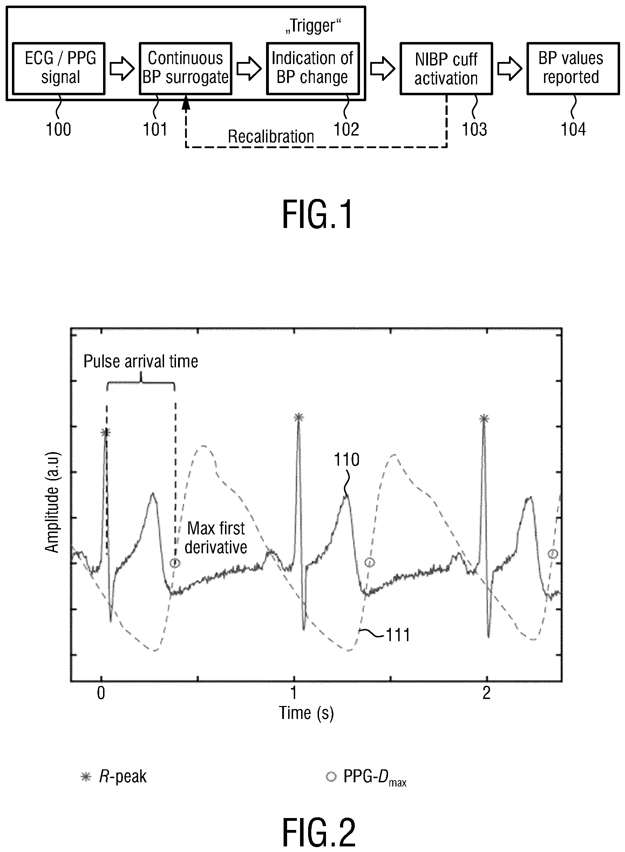

The present invention focuses on the pulse wave velocity methodology towards unobtrusive ABP measurement, since sensor embodiments are small and light-weight and offer comfortable quasi-continuous and non-invasive ABP measurements. An embodiment of the concept is schematically depicted in Fig. 1. In this embodiment ECG and PPG signal are obtained (block 100), e.g. received or retrieved from the respective sensors (which acquire these signals) or from a buffer or memory (where these signal are buffered or stored), and used for continuous BP surrogate determination (block 101). This BP surrogate can then be used as indication (block 102) of a BP change. From time to time, or when a substantial BP change is indicated (which may be used as a trigger) the NIBP cuff may be activated (block 103) to perform a recalibration of the BP surrogate and to obtain a BP reading by an oscillometry approach, e.g. an accepted standard oscillometry approach. BP determinations made by use of the cuff inflation and by use of the BP surrogate may be reported (block 104).

This concept may not need any additional sensors, but can use available sensors, can be implemented in software only and has a low barrier for clinical acceptance. Benefits are earlier interventions through earlier detection of BP changes, reduced complications through reduced use of A-lines, and improved medication titration because of better tracking of BP response.

An embodiment of the presented approach refers to the use of PAT as BP surrogate. PAT is defined as time interval between the peak of the R wave of the ECG and the onset of a peripheral plethysmographic pulse. It has been intensively investigated as surrogate measure of blood pressure and vessel stiffness. PAT is the sum of the pre-ejection period (PEP) and PTT. PEP refers to the time needed for the iso-volumetric ventricle contraction up to the aortic valve opening (AVO) while PTT is the true transit time of the pressure pulse along the arterial wall over a long non-homogeneous vascular path. Only PTT does relate to the pressure-dependent arterial wave propagation modelled by the Moens-Korteweg equation. PEP is a varying additive delay sensitive to stress, emotion and physical effort. Major advantage of PAT vs. PTT is that only one location sensitive transducer (e.g. a PPG sensor) needs to be placed accurately.

Fig. 2 shows a diagram (taken from Shao, J.,

et al., An Optimization Study of Estimating Blood Pressure Models Based on Pulse Arrival Time for Continuous Monitoring) of an ECG signal 110 and a PPG signal 111 in which PAT is indicated as the time delay between the R-peak in the ECG signal and a characteristic point of a peripheral PPG signal. In this example the maximum derivative ($D_{max}$) is selected as characteristic point.

A system using PAT to continuously estimate BP in between NIBP measurements does not need any additional devices (hardware), assuming that time-synchronized PPG and ECG sensors (and/or corresponding signals) are available, and therefore, represents a software-only approach to continuous BP estimation in clinical settings.

PAT changes are inversely related to BP changes: if BP increases, the transmural pressure in the arteries increases, leading to a decrease of the arterial wall compliance, which results in faster propagation time of the blood pulse wave and therefore decreased time until arrival of the pulse wave at the periphery. Vice versa, if BP decreases, PAT increases. The exact transfer function from BP to PAT depends on many factors, such as the patient's individual arterial properties and geometries, the heart's pre-ejection period (PEP), and others. Furthermore, the transfer function is not static, but varies with time, when the patient's hemodynamic status, i.e. the arterial properties (e.g. arterial smooth muscle or PEP), changes.

However, the transfer function of BP and PAT can be modelled by rather simple models (e.g. proportional linear, logarithmic, inverse, inverse square, and others) with e.g. two or three parameters. For example, considering a simple linear model ($BP = m_1 \cdot PAT + m_2$), the two patient-specific parameters are an offset parameter $m_2$ and a sensitivity parameter (slope) $m_1$. In order to apply the transfer function in practice to continuously estimate BP from continuous PAT values, the patient-specific parameters need to be 'learned'. This is called calibration. Furthermore, changes in these parameters are preferably tracked, i.e., calibration is preferably repeated when the model parameters (i.e. the calibration parameters) are changing. This is called re-calibration.

Typically, calibration of a model is done via parameter regression. I.e., if multiple (BP, PAT) data pairs are available, the model curve can be fitted to the data points by means of applying some optimality criterion (e.g. least squares), yielding optimal values for the model parameters based on the provided data. The larger the range of BP and PAT values is, the better (more accurate) are the determined parameters.

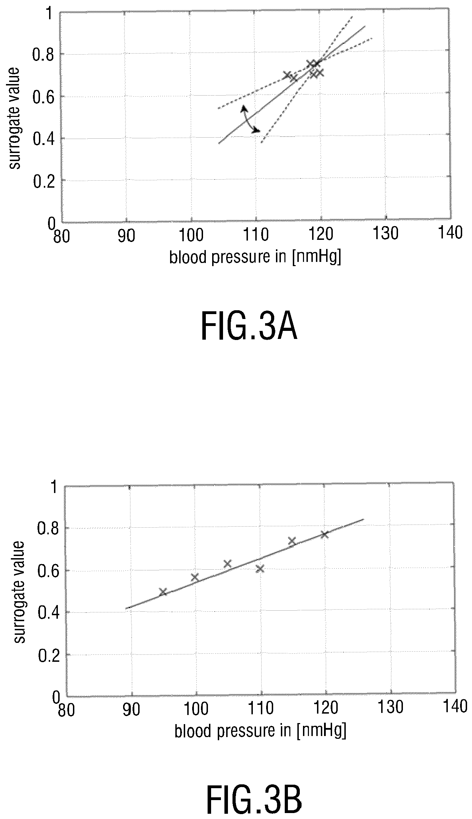

Fig. 3 shows an illustration of robust versus non-robust regression in case of a linear model, in particular diagrams of a linear model for a surrogate fitted to data pairs of blood pressure and surrogate values. Two parameters, offset and slope (or sensitivity parameter), are determined via regression. In the diagram shown in Fig. 3A the regression is not robust, as the data pair values (indicated by x) are too similar. In contrast, the regression shown in the diagram of Fig. 3B is very robust, as the available data pair values span over a larger range.

In practice, achieving robust calibration of the model is a key challenge to be able to reliably use a BP surrogate for unobtrusive determination and monitoring of a subject's BP. Conducting a regression with multiple NIBP and PAT measurements obtained in a short time period suffers from the issue that the resulting curve fit will be bad, since the measured NIBP and PAT values are too similar. To improve the curve fit, the blood pressure level of the patient would need to be changed, which is typically infeasible without altering stiffness of the arterial wall, may cause harm to the patient and is unpractical.

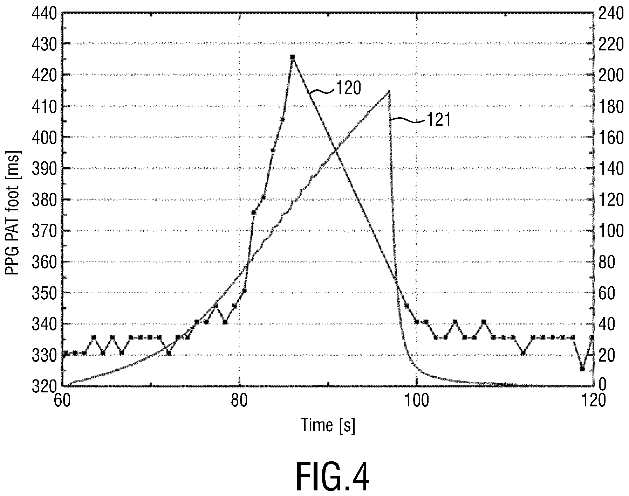

Further, because of the reduced transmural pressure for the artery segment under the cuff, an increase in PAT can be measured in relation of the applied cuff pressure. This is illustrated in Fig. 4 showing a diagram of an example of induced PAT increase 120 during a cuff-inflation 121. The cuff pressure modulates the artery transmural pressure. PAT values are obtained for every beat in synchrony with the cuff pressure. More precisely, this sequence applies to the artery segment with the cuff on top. To infer BP, a scaling factor may need to be incorporated to scale for the whole artery segment where the distal pulse sensor is located.

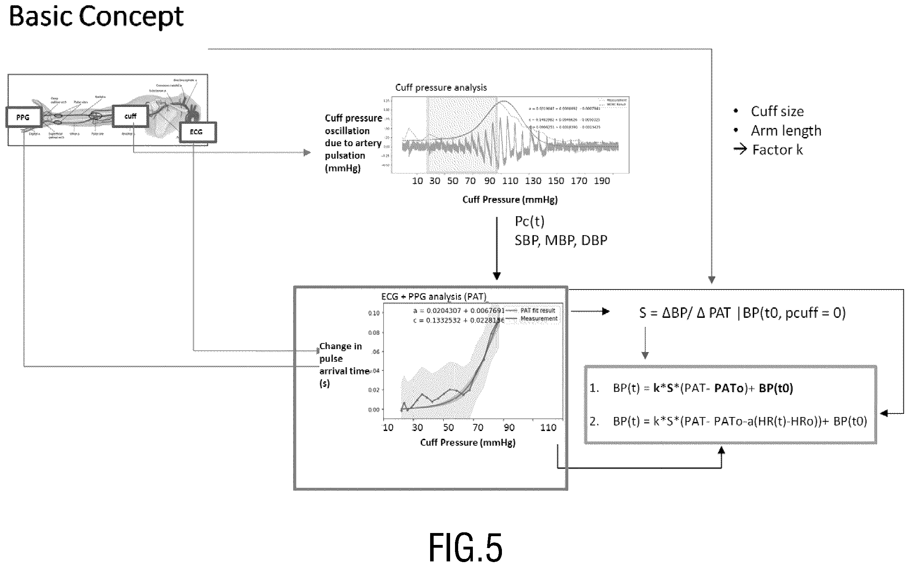

Using the obtained pairs of PATs, the synchronously acquired cuff pressure and the BP values a PAT/BP sensitivity parameter S can be derived, which enables to track BP. This is illustrated in Fig. 5 showing a schematic diagram illustrating the general concept of calibration. During cuff inflation a PAT increase is induced, from which together with the synchronously acquired cuff pressure and the derived BP (via oscillometry) the sensitivity parameter S is derived to track BP. The subject-specific scaling factor in the equations is represented as k. Two examples of mathematical models using the inferred sensitivity parameter S to track BP are shown, which may be used according to embodiments of the present disclosure.

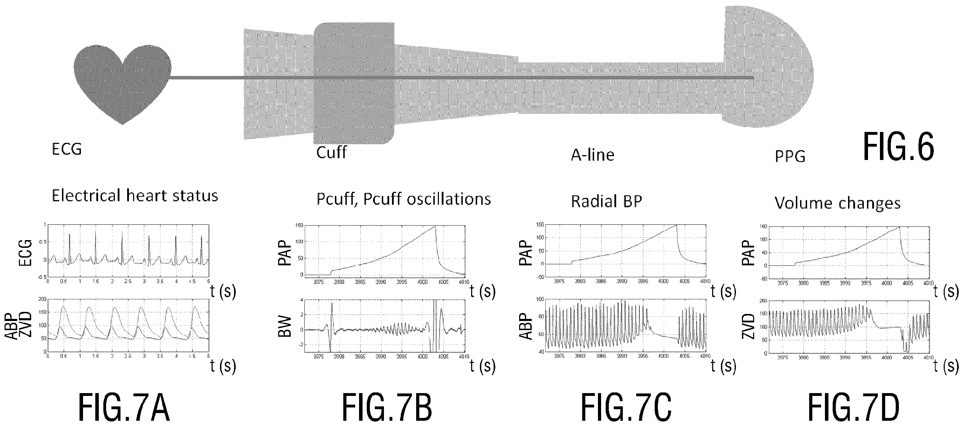

Fig. 6 shows a schematic diagram of an exemplary setup used in the general concept to obtain invasively measured BP signals acquired peripheral to the cuff. Hereby, it shall be noted that the invasive BP measurement is mainly shown here to clarify the BP changes distal to the cuff. New insights and understanding in the physiological processes were found with impact on the calibration process and underlying assumptions for robust and accurate regression of the sensitivity parameter S. It has particularly been found that changes in PAT during cuff pressurization are distorted by dynamic filling effects in the lower arm due to venous occlusion (which affects arterial blood pressure and causes the disturbing effect) and the venous capacity to store blood in the arm segment. This finding has impact on the parameter regression, which needs to be addressed for the inference of the sensitivity parameter S reliably.

Fig. 7 shows diagrams of signals measured by the setup shown in Fig. 6. Fig. 7A shows an ECG signal. Fig. 7B shows a cuff signal. Fig. 7C shows radial BP/A-line (representing the invasive BP signal). Fig. 7D shows a PPG signal.

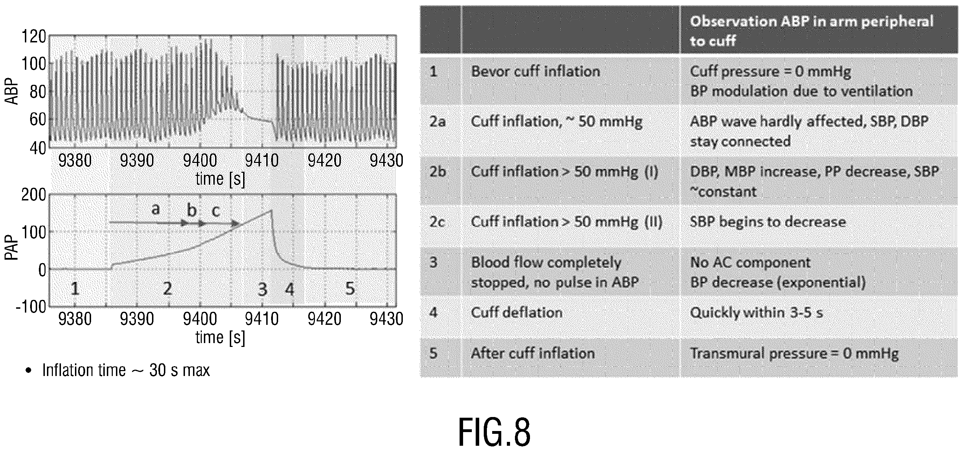

The current calibration approach (applying cuff pressure to induce PAT changes under the cuff) is affected by non-linear dynamic filling effects in the peripheral arterial tree of the arm. Observed processes can be subdivided into various phases with impact on the mathematical modelling functions and consequences for included/excluded data pairs. Fig. 8 shows an analysis of the inflation processes on the blood pressure measured peripherally behind the cuff. Various processes can be identified with impact on PAT. Basic assumption for a successful calibration not dependent on complicated model assumption is a constant peripheral blood pressure in the peripheral arm, which appears to be only present at lower cuff pressures.

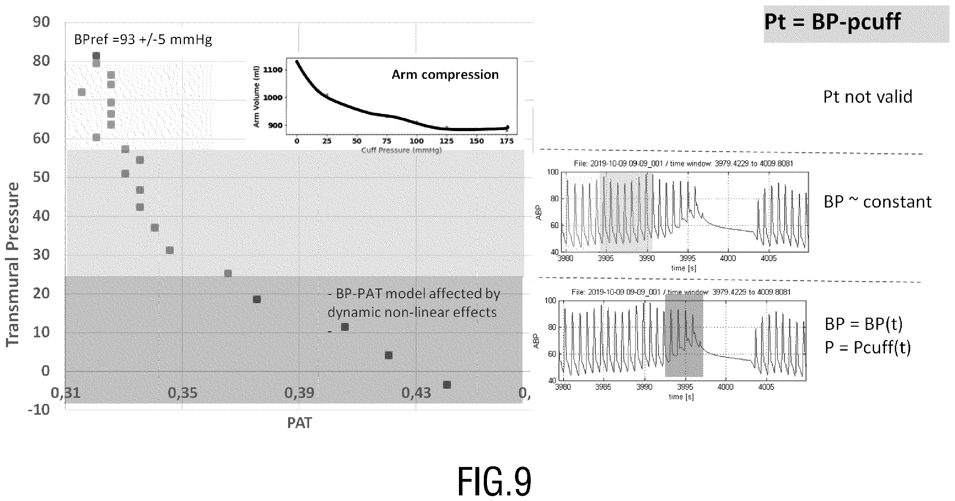

These findings determine different regions in a diagram of transmural blood pressure (SBP- Pcuff; on the y-axis) vs. measured PAT (on the x-axis) as shown in Fig. 9. A typical increase of PAT during cuff inflation is observed, where at least three zones A, B and C can be distinguished. In zone A the arm is compressed at lower cuff pressures and large arm volume changes occur. At this stage peripheral BP is unaltered by the cuff inflation. In zone B the arterial peripheral blood pressure is still unaffected by the cuff inflation. In zone C non-linear dynamic processes are observed in the peripheral blood pressure signal. Zone C is associated with a cuff pressure region, which should not be included in the calibration procedure, since the blood pressure in the peripheral arm changes.

However, regression models assume a constant BP to have a defined transmural pressure during inflation for a specific moment in a pulse phase (e.g. for systolic, mean, diastolic).

Transmural Pressure is defined as: $P_{trans}(t) = P_{arterial}(t) - P_{cuff}(t)$ The key condition for reliable regression during cuff inflation/deflation is that the time dependence $P_{trans}(t)$ must depend on the time-dependence of $P_{cuff}(t)$ only requiring $P_{arterial} = const$ (for a specific moment in a pulse). One implication is that the internal arterial pressure should not be affected by cuff inflation for reliable regression (explained in depth in Fig. 9).

One exemplary regression approach as described in US 2010/160798 A1 includes PAT values only when the cuff pressure is higher than DBP. However, as shown in Fig. 9 and indicated as zone C, at these cuff pressures the blood pressure in the peripheral arm is altered vs. BP at zero cuff pressure and it changes as response to cuff inflation. The calibration concept disclosed in US 2010/160798 A1 should thus be improved, if applicable in the context of the present disclosure at all. Taking into account the findings of the present invention, complicated non-linear regression models depending on vascular resistance, artery closure effects, venous occlusion and pulse distortion effects may be applied at cuff pressure higher than diastolic blood pressure. Such parameters are typically not accessible. In order to address these challenges, a different strategy for the calibration method is required.

The impact on blood pressure monitoring is that inaccurate BP calibration results in unreliable BP inference leading to either false alarms or late and delayed interventions with associated impact on patient outcome. The present invention deals with a solution, which in contrast to state of the art includes PAT as calibration data only when well-defined criteria are valid.

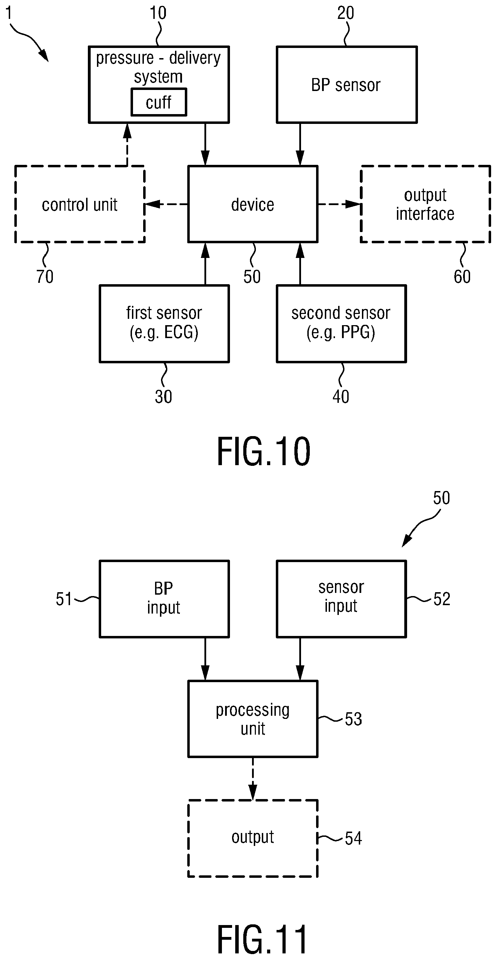

Fig. 10 shows a diagram of an embodiment of a system 1 for calibrating a BP surrogate for use in monitoring a subject's blood pressure according to the present invention.

The system 1 comprises a pressure-delivery system 10 comprising a cuff 11 configured to be attached to the subject's body part and to deliver a pressure to the subject's body part by inflating the cuff. Such a pressure-delivery system 10 comprising a cuff 11 that can be attached to an extremity of the subject, in particular the upper arm, upper leg or wrist, is generally known in the art of NIBP measurement. Generally, it comprises a pressure generating unit, e.g. a pump or pressure reservoir, that is configured to inflate the cuff 11, a valve that is configured to deflate the cuff 11, and a processor that is configured to control the pressure generating unit and the valve and to determine the subject's blood pressure based on the measured cuff pressure. A user interface may be provided, e.g. comprising one or more of a display, keypad, loudspeaker and touchpad, to issue the measured BP values, e.g. in visible and/or audible form.

The system 1 further comprises a pressure sensor 20 configured to acquire time-dependent cuff pressure values during inflation of a cuff of the pressure-delivery system attached to the subject's body part and to acquire a BP measurement value. Typically, the pressure sensor 20 is not directly attached to the subject's body part, but the cuff 11 is attached to the body part and the pressure sensor 20 is attached (at the pressure delivery system) to an air tube that comes from the cuff 11. The pressure sensor 20, e.g. in the form of a pressure transducer, may thus be integrated into the cuff and may be implemented by a conventional BP sensor. Other means to apply an external pressure and to detect cuff pressure, such as a Shellcuff design, are possible as well.

The system 1 further comprises a first sensor 30 configured be attached to a first site of the subject's body and to acquire a first time-dependent sensor signal related to the subject's heartbeat during the inflation of the cuff. The first sensor 30 may be an ECG sensor for acquiring an ECG signal. The first site may e.g. be the subject's chest or torso at which ECG electrodes are preferably attached for ECG measurements.

The system 1 further comprises a second sensor 40 configured be attached to a second site of the subject's body and to acquire a second time-dependent sensor signal related to the subject's heartbeat acquiring signals modulated because of the inflation of the cuff. The second sensor 40 may be a PPG sensor, e.g. a contact PPG sensor (such as a pulse oximetry sensor comprising one or more LEDs emitting light in the visible and/or infrared range, e.g. red and infrared light) or a remote PPG sensor (such as a camera or photo detector, as e.g. known from Verkruysse et al., "Remote plethysmographic imaging using ambient light", Optics Express, 16(26), 22 December 2008, pp. 21434-21445). PPG generally refers to the optical measurement of volume changes of an organ or a body part and in particular to the detection of volume changes due to a cardio-vascular pulse wave traveling through the body of a subject with every heartbeat. Radiation reflected by or transmitted through a skin region of the subject can be detected. The second site may e.g. be the subject's hand or finger at which a contact PPG sensor may be attached or which may be monitored by a remote PPG sensor. The second site is generally a site peripheral to the first site. For instance, if the cuff is attached to the upper left arm, the second site may be the left hand or a finger of the left hand to which the second sensor is attached or which is monitored by the second sensor.

The first and second signals are generally acquired and sampled synchronously. Other signals than ECG and PPG signals that are related to the subject's heart rate and allow the computation of PAT and/or PTT may be used in addition or as alternative, such as a sensor detecting heart sound, a sensor detecting chest vibrations, etc.

The system 1 further comprises a device 50 disclosed herein and described in the following for computing a calibration parameter for calibrating a BP surrogate from the BP measurement value, the time-dependent cuff pressure values and the first and second time-dependent sensor signals. Further, the device 50 may optionally be configured to determine and monitor BP of the subject by use of the BP surrogate.

The system 1 may further optionally comprise an output interface 60 configured to issue any determined information, such as BP values of the subject determined and monitored by use of the BP surrogate. The output interface 60 may generally be any means that outputs information in visual or audible form, e.g. in text form, as image or diagram, as sound or spoken words, etc. For instance, the output interface 60 may be a display, a loudspeaker, a touchscreen, a computer monitor, the screen of a smartphone or tablet, etc.

The system 1 may further optionally comprise a control unit 70 that is configured to control the pressure-delivery system 10, e.g. based on a control signal provided by the device 50 or computed by the system 1. The control unit 70 may be part of the device 50 or may be an external entity.

Fig. 11 shows a diagram of an embodiment of a device 50 for calibrating a BP surrogate for use in monitoring a subject's blood pressure according to the present invention.

The device 50 comprises a BP input 51 configured to obtain time-dependent cuff pressure values during inflation of a cuff 11 of a pressure-delivery system 10 attached to a subject's body part and to obtain a BP measurement value. The device 50 further comprises a sensor input 52 configured to obtain a first time-dependent sensor signal and a second time-dependent sensor signal, both related to the subject's heartbeat and measured at different sites of the subject's body during the inflation of the cuff. The BP input 51 and the sensor input 52 may be directly coupled or connected to the cuff 11 and the first and second sensors 30, 40 or may obtain (i.e. retrieve or receive) these signals from a storage, buffer, network, or bus, etc. The inputs 51 and 52 may thus e.g. be (wired or wireless) communication interfaces or data interfaces, such as a Bluetooth interface, WiFi interface, LAN interface, HDMI interface, direct cable connect, or any other suitable interface allowing signal transfer to the device 50.

The device 50 further comprises a processing unit 53. The processing unit 53 may be any kind of means configured to process the signals and determine calibration parameter for calibration of the BP surrogate. Further, it may be configured to determine and monitor BP of the subject by use of the BP surrogate. It may be implemented in software and/or hardware, e.g. as a programmed processor or computer or app on a user device such as a smartphone, smartwatch, tablet, laptop, PC, workstation, etc.

The device 50 may further comprise an output 54 configured to output any determined information. The output 54 may generally be any interface that provides the determined information, e.g. transmits it to another device or provides it for retrieval by another device (e.g. a smartphone, computer, tablet, etc.). It may thus generally be any (wired or wireless) communication or data interface.

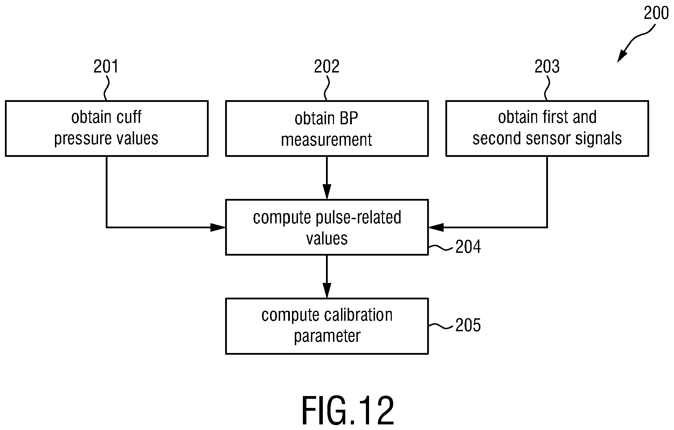

Fig. 12 shows a diagram of an embodiment of a method 200 according to the present invention. The steps of the method 200 may be carried out by the device 50, wherein the main steps of the method are carried out by the processing unit 53. The method may be implemented as computer program running on a computer or processor.

In a first step 201 time-dependent cuff pressure values during inflation of a cuff of a pressure-delivery system attached to a subject's body part are obtained. In a second step 202 a BP measurement value is obtained. In a third step 203 a first time-dependent sensor signal and a second time-dependent sensor signal, both related to the subject's heartbeat and measured at different sites of the subject's body during the inflation of the cuff, are obtained. It shall be noted that the order of the steps 201 to 203 does not indicate a chronological order, but these steps may be carried out in any chronological order. Preferably, steps 201 and 203 are carried out simultaneously, and step 202 is carried out in advance or after steps 201 and 203.

In a fourth step 204, during inflation of the cuff, pulse-related values are computed from the first and second time-dependent sensor signals using a first feature in the first time-dependent sensor signal and a second feature in the second time-dependent sensor signal, the pulse-related values being PAT values or PTT values. PAT or PTT values may be extracted via appropriate feature extraction techniques from the first and second signals (e.g. ECG and PPG signals). Other features may also be derived from the single signals such as heart rate, PPG morphology features, etc.

In a fifth step 205 a calibration parameter for a BP surrogate is computed from the BP measurement value and pairs of pulse-related values and corresponding cuff pressure values, a pair comprising a pulse-related value and a corresponding, temporally related cuff pressure value (in particular, values of the same time instant), wherein only pairs of pulse-related values and corresponding cuff pressure values are used for computing the BP surrogate for which one or more predetermined conditions with respect to BP and/or cuff pressure are fulfilled. For these conditions (or criteria) various options exist as explained below.

Thus, according to a first main aspect of the present invention a BP surrogate can be calibrated and BP can be tracked continuously thereafter. Criteria are used in an embodiment to include cuff pressure ranges during inflation to reliably calibrate BP surrogates from cuff-pressure / BP surrogate data pairs where transmural pressure is well defined as function of applied cuff pressure with $P_{arterial}$ for $P_{cuff} > 0$ is not altered versus $P_{arterial}$ for $P_{cuff} = 0$. Conventionally, calibration requires a complete cuff inflation. For PAT-based surrogate calibration from a continuous inflation-based NIBP measurement, as used in an embodiment, only PAT values induced by cuff pressure levels are utilized given by well-defined criteria fulfilling physiological constraints. Thus, certain data (the data in zone C in Fig. 9) are discarded according to an embodiment. Features in PPG signals may be used as indication of the violation of underlying assumption for the applied regression model, e.g. a change in the pulse pressure in the peripheral arterial segment. Features in the oscillation envelope may be used as indication of the violation of underlying assumption for the applied regression model, e.g. a blood pressure range. Thus, a reliable calibration of PAT as BP pressure surrogate of a patient featuring cuff inflation processes using dedicated procedures and methods is possible.

In the following explanation of methods for BP calibration via regression of BP surrogate / cuff-pressure pairs PAT will be used as BP surrogate. An embodiment is based on the inclusion of data for PAT data for pressure cuff values only for cuff pressures as long as the peripheral blood pressure (i.e., the cuff pressure in the arm behind the cuff, if the cuff is arranged at the upper arm) can be assumed constant, e.g. varies by less than 5 or 10 %.

Full cuff inflation with BP inference may apply oscillometry for BP inference of SBP, DBP and MBP, which typically requires a cuff inflation above systolic BP: Different criteria can be used to select which pairs of PAT values and cuff pressure values to use for determining the calibration parameter. For instance, one or both of the following criteria may be used:

- Criterion 1: PAT / $P_{cuff}$ values only for $P_{cuff}$ < DBP or MBP;
- Criterion 2: PAT / $P_{cuff}$ values for $p_{min}$ < $P_{cuff}$ < DBP, e.g. $p_{min}$ = 20 mmHg.

$p_{min}$ can be defined with respect to observed tissue compression effects (Fig. 7). At cuff pressures where tissue undergoes significant changes in volume, transmural pressure across the artery might be more difficult to be defined reliably.

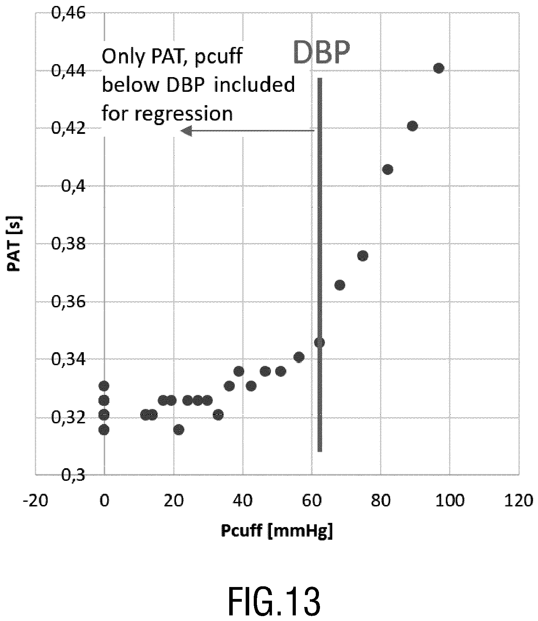

Data acquired during a complete cuff inflation process are shown in the diagram depicted in Fig. 13 as dots points in a plot of PAT versus $P_{cuff}$. By the vertical line DBP it is indicated up to which $P_{cuff}$ value pairs of PAT values (i.e., pulse related values) and cuff pressure values are used for the determination of the calibration parameter.

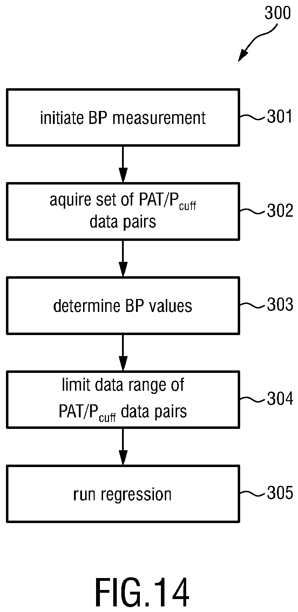

Fig. 14 shows a flow chart of an embodiment of a method 300 using this aspect of the present invention. In a first step 301, a BP measurement is initiated. In a second step 302, a complete set of PAT / $P_{cuff}$ data pairs is acquired during a complete cuff inflation process. In a third step 303, BP values are determined via standard methods, such as systolic blood pressure, diastolic blood pressure and mean blood pressure. In a fourth step 304, data range of PAT / $P_{cuff}$ data pairs is limited according to a predetermined criterion, e.g. according to criterion 1 or 2. In a fifth step 305, a regression is run on acquired (and limited) data pairs. Finally, in a sixth step 306, BP is inferred from calibrated PAT data.

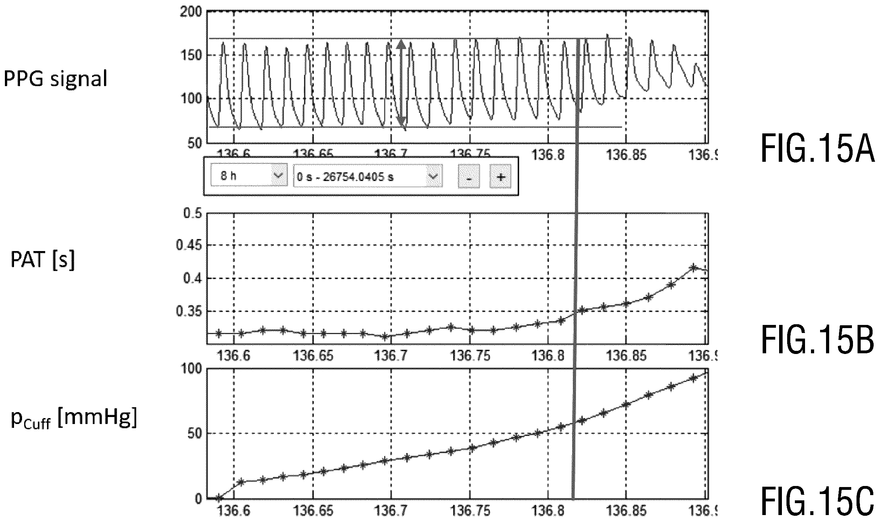

In another embodiment cuff pressure is used as long as e.g. SBP in the peripheral arm (if the cuff is attached to the upper arm) remains constant. Fig. 15 shows diagrams that illustrate that $P_{cuff}$ / PAT value pairs are only used as long as a feature in the PPG signal indicates no pulse pressure change in the peripheral arm blood pressure. Fig. 15A shows PPG signal behavior during cuff inflation. Fig. 15B shows PAT increase during inflation. Fig. 15C shows cuff pressure during inflation. The change in PPG amplitude indicates a change in pulse pressure PP in the peripheral arterial segment and is used as criterion to define maximum PAT / $P_{cuff}$ pairs to be included in regression / calibration. The PPG amplitude is one example, where the beginning of a PPG amplitude decrease indicates the maximum pressure regression data should be used.

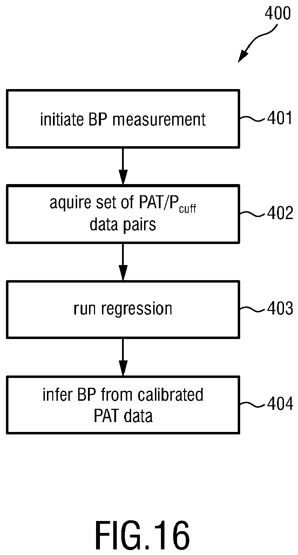

Fig. 16 shows a flow chart of an embodiment of a method 400 using this aspect of the present invention, in particular the use of PPG amplitude change as criterion. In a first step 401 a BP measurement is initiated. In a second step 402 a set of PAT / $P_{cuff}$ data pairs is acquired during a cuff inflation process as long as the PPG amplitude is constant (or changes less than a given %age, e.g. 5 or 10 %). In a third step 403 a regression is run on acquired data; hereby, BP e.g. from a previous complete inflation is used in this regression. Finally, in a fourth step 404, BP is inferred from calibrated PAT data.

As alternative or in addition to the criteria mentioned above, other criteria may be used. Exemplary criteria are: i) Definition of a maximum change in PAT during inflation e.g. include PAT / $P_{cuff}$ values for which PAT-$PAT_{ref}$ < maximum; and ii) observations of the behavior of PAT changes, e.g. the slope of PAT.

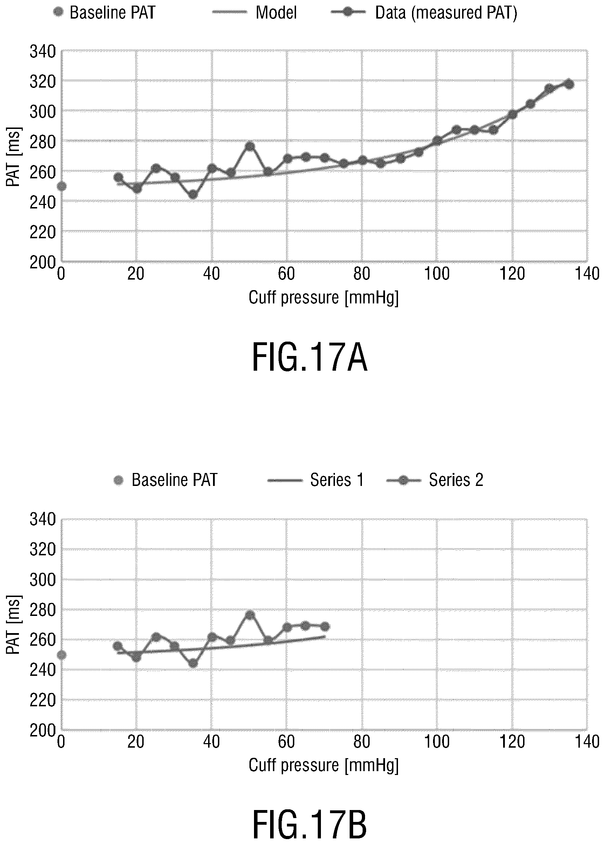

The regression of model parameters is generally based on multiple data pairs: measured PAT values and corresponding cuff pressure values. This is illustrated in the diagrams depicted in Fig.17 showing an example for fitting a parameterized model to measured data pairs (cuff pressure, PAT) that are obtained during cuff inflation. Fig. 17A shows a diagram of a full inflation, whereas Fig. 17B shows a diagram using only low cuff pressure levels for regression leading to a regression that is less robust. As explained above, PAT values can be measured on a beat-by-beat basis. Each time a new pulse is detected at the peripheral sensor, the corresponding PAT can be calculated, and the corresponding cuff pressure is measured. In other words, the number of data pairs that can be used for parameter regression is equal to the number of pulses detected during cuff inflation.

The measured PAT values may, however, be associated with a measurement error. Therefore, the quality (i.e. accuracy) of the parameter regression increases the more data pairs can be used for conducting the actual regression. Therefore, using only the lower part of the curve for parameter regression, i.e. PAT and cuff pressure data pairs up to a certain cuff pressure level, as explained above in an embodiment, may be further improved with respect to the quality of parameter regression. For instance, the overall number of data pairs that can be used for parameter regression is reduced if only the lower part of the curve is used. Further, the signal-to-noise ratio in the regression curve may be reduced. The absolute error in PAT measurement is independent of the applied cuff pressure. However, the desired signal component for parameter regression is the change in PAT from baseline PAT during cuff inflation, not the absolute PAT value. Baseline PAT is the PAT without cuff pressure being applied. Given this, the relative PAT measurement error increases if only low cuff pressure levels are considered, because the change in PAT is low for low cuff pressure levels.

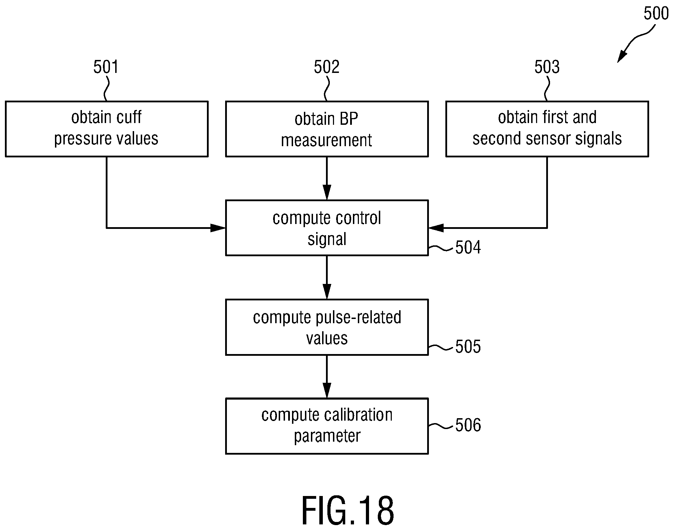

Hence, according to a second main aspect of the present invention a system and device may be used generally having the same units as explained above with respect to the system and device shown in Figs. 10 and 11. The processing unit 53 and the method performed by the processing unit 53 are, however, configured differently. Fig. 18 shows a diagram of an embodiment of a method 500 according to the present invention. The steps of the method 500 may be carried out by the device 50, wherein the main steps of the method are carried out by the processing unit 53. The method may be implemented as computer program running on a computer or processor.

In a first step 501 time-dependent cuff pressure values during inflation of a cuff of a pressure-delivery system attached to a subject's body part are obtained. In a second step 502 a BP measurement value is obtained. In a third step 503 a first time-dependent sensor signal and a second time-dependent sensor signal, both related to the subject's heartbeat and measured at different sites of the subject's body during the inflation of the cuff, are obtained.

In a fourth step 504, a control signal for controlling the pressure-delivery system is computed to repeatedly inflate its cuff in partial inflations up to a cuff pressure below the subject's systolic BP.

In a fifth step 505 pulse-related values are computed from the first and second time-dependent sensor signals, measured during the repeated partial inflations of the cuff, using a first feature in the first time-dependent sensor signal and a second feature in the second time-dependent sensor signal, the pulse-related values being PAT values or PTT values.

In a sixth step 506 a calibration parameter for a BP surrogate is computed from the BP measurement value and pairs of the pulse-related values and corresponding cuff pressure values, computed from the first and second time-dependent sensor signals measured during the repeated partial inflations of the cuff, a pair comprising a pulse-related value and a corresponding cuff pressure value of the same time instant (or at least temporally related values).

Thus, in an exemplary embodiment pump control is configured to repeatedly apply a short increasing pressure profile (pressure ramps) ranging from 0 mmHg cuff pressure to approximately diastolic pressure level or until a pulse amplitude change in the PPG signal is detected, thereby recording cuff pressure and PAT. The processing may conduct parameter regression per applied pressure ramp and average the obtained regression parameters to improve the overall calibration accuracy.

The embodiment explained with reference to Fig. 18 is based on the idea that only low cuff pressure levels should be used for calibration. Repeated sampling of those relevant cuff pressure levels is feasible in a short enough time period, such that the BP level is stable and does not change, which would distort the calibration process. Furthermore, repeated inflation ramps even without a pause in between are enabled by low maximum pressure. There is no need to wait for tissue and arteries to recover from high pressurization (i.e. there is no hysteresis effects).

Another advantage is that cuff pressure levels below diastolic pressure are very unobtrusive for the patient, particularly for awake patients, resulting in less discomfort and less stress to skin than full inflation ramps up to supra-systolic levels.

Still another advantage of such an embodiment is that it does not pose extra requirements on the pressure pump, that is integrated into a NIBP device. An alternative proposal to achieve the same effect by means of a single slow pressure ramp from 0 mmHg to diastolic pressure level would require a pump that can achieve very small air flow at low pressure levels, which would represent a significant challenge to the pump design and is currently not supported by standard pumps used in NIBP devices.

This idea does not apply only to systems using PAT measured by ECG and PPG sensors, but more generally to systems with alternative methods for measuring pulse propagation delay (e.g. based on differential pulse transit time) and with a pressurizable cuff being applied within the propagation path of considered pulse waves.

In the following embodiments of the second aspect to achieve improved BP surrogate calibration via repeated partial inflations are explained. In these embodiments PAT values are inferred on a beat-by-beat basis using the PPG sensor and the ECG sensor.

In an embodiment a baseline value $PAT_{base}$ for PAT is obtained by averaging the PAT measurements over a certain period. That period can be predetermined and fixed or set by user, e.g. to 20s or 15s or any other sufficiently long period in time to sufficiently suppress unwanted fluctuations caused by e.g. respiration (typical frequency of 0.1-0.5 Hz), Meyer waves (typical frequency of 0.1 Hz), or other measurement noise.

By activating the pump of the NIBP device a first (partial) inflation ramp up to a certain sub-systolic level is conducted. This can be diastolic pressure (known from the last NIBP measurement), some specified sub-diastolic level, a certain fixed cuff pressure level (e.g. 60 mmHg or 40 mmHg), or a pressure level where PAT has exceeded a certain threshold compared to $PAT_{base}$ or a pre-defined amplitude change of the PPG amplitude is detected. The inflation speed may be set to a fixed inflation speed, such as e.g. 10 mmHg/s, or otherwise chosen, e.g. dependent on the heart rate to guarantee a certain minimum number of heart beats within the inflation ramp. During cuff inflation, cuff pressure $P_{cuff}$ is measured at the same time instances where PAT values are measured. The difference in PAT compared to baseline $PAT_{base}$, denoted as $\Delta PAT$, is computed for the PAT values during cuff inflation.

The measured data pairs ($\Delta PAT$, $P_{cuff}$) are used for conducting a parameter regression (curve fitting) of the unknown model parameters $m_1$, $m_2$, ..., $m_N$ of the parameterized model function f, that models the relation between a change in PAT and a change in blood pressure $\Delta BP$ around an operating point. During cuff inflation the change in blood pressure is $\Delta BP = - P_{cuff}$, as the blood pressure in the arterial segment of length $L_{cuff}$ under the cuff is reduced by $P_{cuff}$ (this explanation applies to the approach described above as well). Therefore, the regression equation is obtained as follows:

$$\Delta\text{PAT} = \text{L}_{\text{cuff}} \cdot \text{f}(-\text{P}_{\text{cuff}}; \text{m}_1, \text{m}_2, \dots, \text{m}_N).$$

As an example, in a simple linear proportional model the number of unknown model parameters would be two ($f = m_1 \cdot \Delta BP + m_2$). A preferred criterion for parameter regression is the least square error, however, parameter regression could be conducted according to different error measures, such as e.g. least absolute error.

In order to improve parameter regression, the cuff is deflated, another partial inflation is conducted, and another parameter regression is performed. This can be repeated multiple times. In preferred embodiment one extra partial inflation is conducted. Subsequently, the obtained regression values for the model parameters are averaged, thereby improving the accuracy of model-parameter calibration. The degree of variation of obtained regression values can be used to decide how many repeated partial inflations shall be conducted. For example, if the obtained regression values after two partial inflations closely align, no further partial inflation is needed.

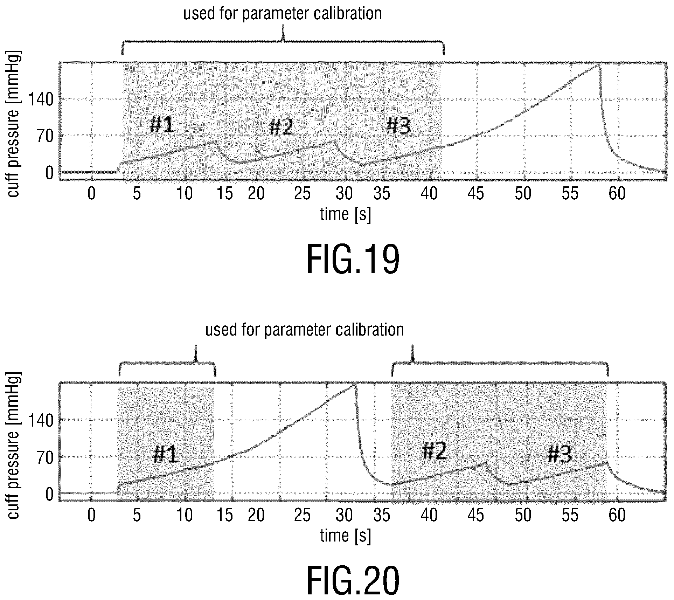

In order to calibrate the resulting continuous BP estimator a NIBP measurement is conducted, yielding a blood pressure reference measurement $BP_{ref}$. This is enabled by continuing the last partial inflation to suprasystolic cuff pressure level, such that a standard inflation-based oscillometric blood pressure measurement can be computed. In other words, the last inflation ramp is a 'full' cuff inflation ramp. Fig. 19 shows a diagram that illustrates the proposed cuff pressure profile at the example of two extra partial inflations (before a full inflation) to improve model parameter calibration before a full inflation is conducted. Finally, the calibrated PAT-based continuous blood pressure estimator is obtained as

$$BP = BP_{ref} + f^{-1}\left(\frac{\Delta\text{PAT}}{L}\right),$$

where $f^{-1}$ denotes the inverse function of f, using the average of the model-parameters obtained by the multiple regressions. L is a population-based parameter, determined by the length of the arterial tree from the heart to the peripheral PPG sensor. L is e.g. inferred via the arm length.

Typically, these steps are repeated from time to time to recalibrate the continuous blood pressure estimator. This can be done according to some regular schedule, or in case significant blood pressure changes occur. In general, the proposed pressure profile of the type shown in Fig. 19, i.e., the pressure profile comprising repeated partial inflations, can be performed whenever an NIBP measurement is triggered automatically or manually.

In an alternative embodiment the partial inflations are not prepended but appended. Fig. 20 shows a diagram that illustrates the proposed cuff pressure profile at the example of two extra partial inflations (after a full inflation). This profile has the advantage that the NIBP value can be displayed sooner compared to the previous embodiment of the pressure profile illustrated in Fig. 19. However, the time distance between the first parameter calibration and the second parameter calibration is longer. If the patient's hemodynamic status is not constant during this time, i.e., if the model parameters are changing, the gain in accuracy by averaging over multiple calibration events is diminished.

In another alternative embodiment improved parameter regression is not obtained by averaging over the regression parameters from multiple partial inflations, but rather by using all obtained data pairs ($\Delta PAT$, $P_{cuff}$) from multiple partial inflations for a single regression. For this option, the accuracy gain is obtained by having more regression data points available for a single regression. Furthermore, instead of predetermining the number of conducted partial inflations, it could rather be determined though threshold comparison of the regression error. If the regression error drops below that threshold, no further partial inflation is needed.

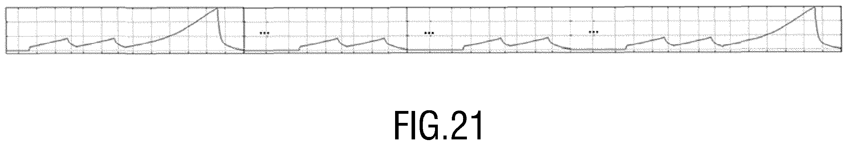

Still another alternative embodiment further provides that after the continuous blood pressure estimator has been calibrated via model-parameter regression and NIBP reference measurement, only partial inflations are conducted, e.g. every 10 minutes. This may be done as explained above. A 'full calibration' with NIBP reference measurement is only conducted if the model-parameter regression results in a relevant change of model parameters $m_1, \dots m_N$, compared to the parameter regression result associated with the last 'full calibration'. If no relevant change is detected, the parameters of the current continuous blood pressure estimator are not updated and no full inflation for NIBP measurement is conducted. Fig. 21 shows a diagram of a pressure profile that provides continual tracking of recalibration need through repeated partial inflations. The advantage of this concept is that it has the potential for long periods of unobtrusive operation. This is advantageous for patients with multiple days of automated NIBP monitoring, e.g. supporting the healing process of ICU patients through improved sleep quality during the night.

The present invention applies to the broad range of applications, e.g. for in-hospital monitoring settings, where NIBP, ECG, and PPG sensors are already available and used. For example, these sensors are virtually used in all settings in the OR and the ICU, but this includes also certain settings in the general ward as well as settings in specialized wards like neurology. More specifically, it allows for measuring beat-to-beat arterial blood pressure non-invasively. Beat-to-beat blood pressure monitoring can provide early detection of hypotensive events, which are associated with adverse patient outcome. Furthermore, it can provide an early warning for hemodynamic instability. The disclosed apparatus and method may also be applied for long-term monitoring, e.g. for 24h BP monitoring or BP monitoring at night during sleep.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

Further embodiments of the claimed system, device and method may be configured as follows:

1. Device as disclosed herein, wherein the processing unit (53) is configured to:

- compute, during inflation of the cuff, pulse-related values from the first and second time-dependent sensor signals using a first feature in the first time-dependent sensor signal and a second feature in the second time-dependent sensor signal, the pulse-related values being pulse arrival time, PAT, values or pulse transit time, PTT, values, and
- compute a calibration parameter for a BP surrogate from the BP measurement value and pairs of pulse-related values and corresponding cuff pressure values, a pair comprising a pulse-related value and a corresponding, temporally related cuff pressure value, wherein only pairs of pulse-related values and corresponding cuff pressure values are used for computing the BP surrogate for which one or more predetermined conditions with respect to BP and/or cuff pressure are fulfilled.

2. Device as defined in embodiment 1, wherein the processing unit (53) is configured to use only pairs of pulse-related values and corresponding cuff pressure values for computing the calibration parameter for the BP surrogate that have been computed from the first and second time-dependent sensor signals measured during a partial inflation of the cuff.

3. Device as defined in embodiment 1 or 2, wherein the processing unit (53) is configured to use only pairs of pulse-related values and corresponding cuff pressure values for computing the calibration parameter for the BP surrogate for which a peripheral BP is substantially constant, in particular varies by less than 10 % or less than 5 %.

4. Device as defined in embodiment 3, wherein the processing unit (53) is configured to determine if the peripheral BP is substantially constant based on the first and/or second time-dependent sensor signal, in particular by determining the period during which an amplitude of the second time-dependent signal is substantially constant, in particular varies by less than 10 % or less than 5 % with respect to a previously acquired average value.

5. Device as defined in any one of the preceding embodiments, wherein the processing unit (53) is configured to use only pairs of pulse-related values and corresponding cuff pressure values for computing the calibration parameter for the BP surrogate for which the cuff pressure value is below the subject's diastolic BP, in particular the subject's latest measured diastolic BP, or below the subject's mean BP, in particular the subject's latest measured mean BP, or below a set threshold cuff pressure.

6. Device as defined in any one of the preceding embodiments, wherein the processing unit (53) is configured to use only pairs of pulse-related values and corresponding cuff pressure values for computing the calibration parameter for the BP surrogate for which the cuff pressure value is in a range between a set minimum cuff pressure and a set maximum cuff pressure, in particular wherein the minimum cuff pressure is set in the range of 10 to 30 mmHg and the maximum cuff pressure is set in the range of 40 to 90 mmHg.

7. Device as defined in any one of the preceding embodiments, wherein the processing unit (53) is configured to use only pairs of pulse-related values and corresponding cuff pressure values for computing the calibration parameter for the BP surrogate for which the pulse-related values are substantially constant, in particular vary by less than 10 %.

8. Device as defined in any one of the preceding embodiments, wherein the processing unit (53) is configured to use only pairs of pulse-related values and corresponding cuff pressure values for computing the calibration parameter for the BP surrogate until the slope of a curve of the pulse-related values over time exceeds a threshold.

9. Device as defined in any one of the preceding embodiments,
wherein the processing unit (53) is compute a control signal for controlling the pressure-delivery system (10) to inflate its cuff (11), in particular to fully inflate the cuff for acquisition of the BP measurement value and to only partly inflate the cuff for acquisition of the first time-dependent sensor signal and the second time-dependent sensor signal.

10. Device as defined in any one of the preceding embodiments,
wherein the first time-dependent sensor signal is an ECG signal and/or the second time-dependent sensor signal is a photoplethysmography, PPG, signal, in particular a contact PPG signal or a remote PPG signal.

11. Device as defined in embodiment 1,
wherein the processing unit (53) is configured to compute the calibration parameter by use of a regression, in particular to compute the calibration parameter as a slope of a dependency of a relation of the blood pressure as a function of the blood pressure surrogate for zero cuff pressure.

12. Device as defined in any one of the preceding embodiments,
wherein the processing unit (53) is configured to determine BP values when no pressure is delivered to the subject's body part by the pressure-delivery system by use of the computed BP surrogate and the measured first and second time-dependent sensor signals measured at different sites of the subject's body when no pressure is delivered to the subject's body part.

13. System for calibrating a blood pressure, BP, surrogate for use in monitoring a subject's blood pressure, the system comprising:

   a pressure-delivery system (10) comprising a cuff (11) configured to be attached to the subject's body part and to deliver a pressure to the subject's body part by inflating the cuff;
   a pressure sensor (20) configured to acquire time-dependent cuff pressure values during inflation of a cuff of the pressure-delivery system attached to the subject's body part and to acquire or infer a BP measurement value;
   a first sensor (30) configured be attached to a first site of the subject's body and to acquire a first time-dependent sensor signal related to the subject's heartbeat during the inflation of the cuff;
   a second sensor (40) configured be attached to a second site of the subject's body and to acquire a second time-dependent sensor signal related to the subject's heartbeat during the inflation of the cuff; and
   a device (50) as disclosed herein for computing a calibration parameter for calibrating a BP surrogate from the BP measurement value, the time-dependent cuff pressure values and the first and second time-dependent sensor signals.

14. Method as disclosed herein comprising:

- computing, during inflation of the cuff, pulse-related values from the first and second time-dependent sensor signals using a first feature in the first time-dependent sensor signal and a second feature in the second time-dependent sensor signal, the pulse-related values being pulse arrival time, PAT, values or pulse transit time, PTT, values, and
- computing a calibration parameter for a BP surrogate from the BP measurement value and pairs of pulse-related values and corresponding cuff pressure values, a pair comprising a pulse-related value and a corresponding, temporally related cuff pressure value, wherein only pairs of pulse-related values and corresponding cuff pressure values are used for computing the BP surrogate for which one or more predetermined conditions with respect to BP and/or cuff pressure are fulfilled.

15. Computer program comprising program code means for causing a computer to carry out the steps of the method as defined in embodiment 14 when said computer program is carried out on the computer.

## Claims

1. Device for calibrating a blood pressure, BP, surrogate for use in monitoring a subject's blood pressure, the device comprising:

   a BP input (51) configured to obtain time-dependent cuff pressure values during inflation of a cuff (11) of a pressure-delivery system (10) attached to a subject's body part and a BP measurement value;
   a sensor input (52) configured to obtain a first time-dependent sensor signal and a second time-dependent sensor signal, both related to the subject's heartbeat and measured at different sites of the subject's body during the inflation of the cuff; and
   a processing unit (53) configured to:

      - compute a control signal for controlling the pressure-delivery system (10) to repeatedly inflate its cuff in partial inflations up to a cuff pressure below the subject's systolic BP,
      - compute pulse-related values from the first and second time-dependent sensor signals, measured during the repeated partial inflations of the cuff, using a first feature in the first time-dependent sensor signal and a second feature in the second time-dependent sensor signal, the pulse-related values being pulse arrival time, PAT, values or pulse transit time, PTT, values, and
- compute a calibration parameter for a BP surrogate from the BP measurement value and pairs of the pulse-related values and corresponding cuff pressure values, computed from the first and second time-dependent sensor signals measured during the repeated partial inflations of the cuff, a pair comprising a pulse-related value and a corresponding, temporally related cuff pressure value.

2.  Device as claimed in claim 1,
    wherein the processing unit (53) is configured to compute the control signal for controlling the pressure-delivery system (10) to inflate its cuff in the partial inflations as long as a peripheral BP is substantially constant, in particular varies by less than 10 percent or less than 5 percent.

3.  Device as claimed in claim 2,
    wherein the processing unit (53) is configured to determine if the peripheral BP is substantially constant based on the first and/or second time-dependent sensor signal, in particular by determining the period during which an amplitude of the second time-dependent signal is substantially constant, in particular varies by less than 10 percent or less than 5 percent with respect to a previously acquired average value.

4.  Device as claimed in claim 1,
    wherein the processing unit (53) is configured to compute the control signal for controlling the pressure-delivery system (10) to inflate its cuff in the partial inflations up to the subject's diastolic BP, in particular the subject's latest measured diastolic BP, up to the subject's mean BP, in particular the subject's latest measured mean BP, or up to a set threshold cuff pressure.

5.  Device as claimed in claim 1,
    wherein the processing unit (53) is configured to compute the control signal for controlling the pressure-delivery system (10) to inflate its cuff in the partial inflations up to a cuff pressure at which the PAT exceeds a PAT threshold, in particular an absolute PAT threshold or a relative PAT threshold compared to a baseline PAT.

6.  Device as claimed in claim 5,
    wherein the processing unit (53) is configured to compute the baseline PAT by averaging PAT measurements over a period, in particular a period in the range of 10 s to 5 min.

7.  Device as claimed in claim 1,
    wherein the processing unit (53) is configured to compute the control signal for controlling the pressure-delivery system (10) to inflate its cuff in the partial inflations up to a cuff pressure at which an amplitude of the second time-dependent signal exceeds an amplitude threshold, in particular an absolute amplitude threshold or a relative amplitude threshold.

8.  Device as claimed in any one of the preceding claims,
    wherein the processing unit (53) is configured to compute the control signal for controlling the pressure-delivery system (10) to inflate its cuff in the partial inflations at a predetermined inflation speed or an inflation speed dependent on one or more of the subject's heart rate, the subject's diastolic BP, the subject's mean BP, and the subject's systolic BP.

9.  Device as claimed in any one of the preceding claims,
    wherein the processing unit (53) is configured to:

    - compute the control signal for controlling the pressure-delivery system (10) to deflate its cuff after each iteration of a partial inflation,
    - i) compute, for each iteration, the one or more calibration parameters from pairs of pulse-related values and corresponding cuff pressure values obtained in the respective iteration using separate regressions, and average the respective calibration parameters computed for the two or more iterations into one or more averaged calibration parameters for use by the BP surrogate, or

    ii) compute the one or more calibration parameters from pairs of pulse-related values and corresponding cuff pressure values obtained in the two or more iterations in a single regression.

10. Device as claimed in any one of the preceding claims,
    wherein the processing unit (53) is configured to compute the control signal for controlling the pressure-delivery system (10) to inflate its cuff in a full inflation beyond the subject's systolic BP before and/or after one or more iterations of partial inflation to obtain a time-dependent BP reference measurement for use in monitoring the subject's BP using the BP surrogate.

11. Device as claimed in any one of the preceding claims,

wherein the processing unit (53) is configured to control how many iterations of partial inflation are conducted based on a comparison of a regression error to a regression error threshold and/or to control if and when a full inflation is conducted according to a fixed or variable schedule or if one or more calibration parameters substantially changed in the last computation, in particular changed by more than 10percent.

12. Device as claimed in any one of the preceding claims,
   wherein the processing unit (53) is configured to determine BP values when no pressure is delivered to the subject's body part by the pressure-delivery system by use of the computed BP surrogate and the measured first and second time-dependent sensor signals measured at different sites of the subject's body when no pressure is delivered to the subject's body part.

13. System for calibrating a blood pressure, BP, surrogate for use in monitoring a subject's blood pressure, the system comprising:

   a pressure-delivery system (10) comprising a cuff (11) configured to be attached to the subject's body part and to deliver a pressure to the subject's body part by inflating the cuff;
   a pressure sensor (20) configured to acquire time-dependent cuff pressure values during inflation of a cuff of the pressure-delivery system attached to the subject's body part and to acquire or infer a BP measurement value;
   a first sensor (30) configured be attached to a first site of the subject's body and to acquire a first time-dependent sensor signal related to the subject's heartbeat during the inflation of the cuff;
   a second sensor (40) configured be attached to a second site of the subject's body and to acquire a second time-dependent sensor signal related to the subject's heartbeat during the inflation of the cuff; and
   a device (50) as claimed in any one of the preceding claims for computing one or more calibration parameters for calibrating a BP surrogate from the BP measurement value, the time-dependent cuff pressure values and the first and second time-dependent sensor signals.

14. Method for calibrating a blood pressure, BP, surrogate for use in monitoring a subject's blood pressure, the method comprising:

   - obtaining time-dependent cuff pressure values during inflation of a cuff (11) of a pressure-delivery system (10) attached to a subject's body part;
   - obtaining a BP measurement value;
   - obtaining a first time-dependent sensor signal and a second time-dependent sensor signal, both related to the subject's heartbeat and measured at different sites of the subject's body during the inflation of the cuff;
   - computing a control signal for controlling the pressure-delivery system (10) to repeatedly inflate its cuff in partial inflations up to a cuff pressure below the subject's systolic BP,
   - computing pulse-related values from the first and second time-dependent sensor signals, measured during the repeated partial inflations of the cuff, using a first feature in the first time-dependent sensor signal and a second feature in the second time-dependent sensor signal, the pulse-related values being pulse arrival time, PAT, values or pulse transit time, PTT, values, and
   - computing a calibration parameter for a BP surrogate from the BP measurement value and pairs of the pulse-related values and corresponding cuff pressure values, computed from the first and second time-dependent sensor signals measured during the repeated partial inflations of the cuff, a pair comprising a pulse-related value and a corresponding, temporally related cuff pressure value of the same time instant.

15. Computer program comprising program code means for causing a computer to carry out the steps of the method as claimed in claim 14 when said computer program is carried out on the computer.

FIG.1

✳ *R*-peak        ○ PPG-$D_{max}$

FIG.2

FIG.3A

FIG.3B

FIG.4

FIG.5

ECG          Cuff          A-line          PPG

FIG.6

Electrical heart status     Pcuff, Pcuff oscillations     Radial BP     Volume changes

FIG.7A        FIG.7B        FIG.7C        FIG.7D

EP 4 173 555 A1

| | | Observation ABP in arm peripheral to cuff |
|---|---|---|
| 1 | Bevor cuff inflation | Cuff pressure = 0 mmHg<br>BP modulation due to ventilation |
| 2a | Cuff inflation, ~ 50 mmHg | ABP wave hardly affected, SBP, DBP stay connected |
| 2b | Cuff inflation > 50 mmHg (I) | DBP, MBP increase, PP decrease, SBP ~constant |
| 2c | Cuff inflation > 50 mmHg (II) | SBP begins to decrease |
| 3 | Blood flow completely stopped, no pulse in ABP | No AC component<br>BP decrease (exponential) |
| 4 | Cuff deflation | Quickly within 3-5 s |
| 5 | After cuff inflation | Transmural pressure = 0 mmHg |

- Inflation time ~ 30 s max

FIG.8

FIG.9

FIG.10

FIG.11

EP 4 173 555 A1

200

201

obtain cuff
pressure values

202

obtain BP
measurement

203

obtain first and
second sensor signals

compute pulse-related
values

204

compute calibration
parameter

205

# FIG.12

FIG.13

300

initiate BP measurement — 301

aquire set of PAT/P$_{cuff}$ data pairs — 302

determine BP values — 303

limit data range of PAT/P$_{cuff}$ data pairs — 304

run regression — 305

# FIG.14

PPG signal

FIG.15A

PAT [s]

FIG.15B

p$_{Cuff}$ [mmHg]

FIG.15C

400

| initiate BP measurement | 401 |

| aquire set of PAT/P$_{cuff}$ data pairs | 402 |

| run regression | 403 |

| infer BP from calibrated PAT data | 404 |

FIG.16

FIG.17A

FIG.17B

500

| 501 | 502 | 503 |
|---|---|---|
| obtain cuff pressure values | obtain BP measurement | obtain first and second sensor signals |

compute control signal — 504

compute pulse-related values — 505

compute calibration parameter — 506

# FIG.18

FIG.19

FIG.20

FIG.21

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | US 2010/160798 A1 (BANET MATT [US] ET AL) 24 June 2010 (2010-06-24) | 1,13-15 | INV. A61B5/021 A61B5/022 A61B5/00 |
| A | * the whole document * | 2-12 | |
| A | J MUEHLSTEFF ET AL: "Cuffless Estimation of Systolic Blood Pressure for Short Effort Bicycle Tests: The Prominent Role of the Pre-Ejection Period", ENGINEERING IN MEDICINE AND BIOLOGY SOCIETY (EMBC), 2013 34TH ANNUAL INTERNATIONAL CONFERENCE OF THE IEEE, vol. 1, 1 August 2006 (2006-08-01), pages 5088-5092, XP055493570, ISSN: 1557-170X, DOI: 10.1109/IEMBS.2006.260275 * page 5090, line 3, last paragraph - line 23; figure 1 * | 2-12 | |
| A | US 2017/367592 A1 (MALTZ JONATHAN S [US]) 28 December 2017 (2017-12-28) * the whole document * | 1-15 | |

TECHNICAL FIELDS SEARCHED (IPC)

A61B

The present search report has been drawn up for all claims

| Place of search | Date of completion of the search | Examiner |
|---|---|---|
| The Hague | 25 March 2022 | Davis, Alan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 21 20 4644

This annex lists the patent family members relating to the patent documents cited in the above-mentioned European search report.
The members are as contained in the European Patent Office EDP file on
The European Patent Office is in no way liable for these particulars which are merely given for the purpose of information.

25-03-2022

| Patent document cited in search report | | | Publication date | Patent family member(s) | | | Publication date |
|---|---|---|---|---|---|---|---|
| US | 2010160798 | A1 | 24-06-2010 | CN | 102811659 | A | 05-12-2012 |
| | | | | CN | 105342591 | A | 24-02-2016 |
| | | | | EP | 2519144 | A1 | 07-11-2012 |
| | | | | HK | 1218613 | A1 | 03-03-2017 |
| | | | | SG | 181970 | A1 | 30-08-2012 |
| | | | | SG | 10201501410U | A | 28-05-2015 |
| | | | | US | 2010160798 | A1 | 24-06-2010 |
| | | | | WO | 2011082341 | A1 | 07-07-2011 |
| US | 2017367592 | A1 | 28-12-2017 | AU | 2016308466 | A1 | 08-03-2018 |
| | | | | CA | 2995472 | | 23-02-2017 |
| | | | | CN | 108135506 | A | 08-06-2018 |
| | | | | EP | 3334336 | A1 | 20-06-2018 |
| | | | | JP | 2018525106 | A | 06-09-2018 |
| | | | | US | 2017042431 | A1 | 16-02-2017 |
| | | | | US | 2017367592 | A1 | 28-12-2017 |
| | | | | US | 2018325389 | A1 | 15-11-2018 |
| | | | | WO | 2017031005 | A1 | 23-02-2017 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2010160798 A1

**Non-patent literature cited in the description**

- **PIELMUS, A. G. ; MIIHLSTEFF, J. ; BRESCH, E. ; GLOS, M. ; JUNGEN, C. ; MIEKE, S. ; ZAUNSEDER, S.** Surrogate based continuous noninvasive blood pressure measurement. *Biomedical Engineering/Biomedizinische Technik,* 2021, vol. 66 (3), 231-245
- **SHAO, J. et al.** *An Optimization Study of Estimating Blood Pressure Models Based on Pulse Arrival Time for Continuous Monitoring*
- **VERKRUYSSE et al.** Remote plethysmographic imaging using ambient light. *Optics Express,* 22 December 2008, vol. 16 (26), 21434-21445